# EUROPÄISCHE PATENTANMELDUNG

(11) **EP 2 881 094 A1**
(43) Veröffentlichungstag der Anmeldung: **10.06.2015**
(21) Anmeldenummer: 13195660.9
(22) Anmeldetag: 04.12.2013
(51) Int. Cl.: A61F 13/06, A61F 13/10, A61F 13/12, A61F 13/14, A41C 3/00

(54) **Therapeutisches Kleidungsstück**

(71) Anmelder: Ranly, Kornelia, 64342 Seeheim-Jugenheim (DE)
(72) Erfinder: Ranly, Kornelia, 64342 Seeheim-Jugenheim (DE)
(74) Vertreter: Tergau & Walkenhorst Patentanwälte - Rechtsanwälte

(57) **Zusammenfassung**

Die Erfindung betrifft ein Kleidungsstück (2), insbesondere ein therapeutisches Kleidungsstück, welches mittels Drucksträngen (8) die manuelle Lymphdrainage durch tragen des Kleidungsstücks nahezu ersetzt und so den Lymphabfluss entscheidend verbessert. Zu diesem Zweck ist erfindungsgemäß vorgesehen, dass auf der der Haut zugewandten Seite, von der Peripherie aus in Richtung der Lymphknotenregionen verlaufend, eine Anzahl von Drucksträngen (8) eingebracht ist.

## Beschreibung

Die Erfindung bezieht sich auf ein therapeutisches Kleidungsstück zum Tragen auf der Haut.

Nicht nur operative Eingriffe an der weiblichen Brust, wie z.B. zur Behandlung von Brustkrebserkrankungen, als auch weitere therapeutische Verfahren, beispielsweise die Bestrahlung der betreffenden Brust, führen vielfach zu Lymphabflussstörungen, die in der Folge eine Schmerzsymptomatik nach sich ziehen, die für die betroffenen Patientinnen zu Einschränkungen im Alltag sowie einer verminderten Lebensqualität führen. Aus Umfragen geht hervor, dass ca. ein Drittel aller Frauen, die sich aufgrund von Brustkrebs einer brusterhaltenden Therapie mit sich anschließender Bestrahlung unterzogen haben, ihre Brust nach der Behandlung permanent spüren, 10% fühlen sich bei ihren täglichen Aufgaben beeinträchtigt. Als Ursache für diese Beschwerden wird häufig eine Störung des Lymphabflusses festgestellt. Die Störung des Lymphabflusses führt in der Folge zu einer Reihe von subchronischen bis chronischen Beschwerden, beispielsweise zu einer schmerzhaften Brustschwellung. Üblicherweise sind die Beschwerden kurz nach dem Aufstehen am wenigsten ausgeprägt und nehmen im Tagesverlauf stetig zu, da sich Gewebsflüssigkeit, insbesondere in einer ptotischen Brust, in den Bereichen der Brust ansammelt, die unterhalb der Brustfalte liegen.

Eine der bekanntesten Therapieformen umfasst die Kombination aus manueller Lymphdrainage, beispielsweise durch spezielle Massagetechniken, und der anschließenden Kompression der Brust mittels entsprechenden Vorrichtungen. Die Massagetechnik unterstützt den Lymphabfluss, während das Anlegen beispielsweise von Kompressionsbandagen eine erneute Ansammlung von Gewebsflüssigkeit in der Brust zwar nicht verhindert, aber immerhin verlangsamt.

Um die Kompression auch im Alltag aufrecht zu erhalten und so die Lymphe daran zu hindern problemlos wieder zurück in das betreffende Gewebe zu fließen, werden medizinische Kleidungsstücke verwendet. Diese medizinischen Kleidungsstücke zeichnen sich im Wesentlichen dadurch aus, dass sie Druck auf die betreffenden Stellen ausüben. Um durch den Druck nicht die Blutversorgung zu unterbrechen, was mitunter beim Sport zu Problemen führt, werden in das Material teilweise Stege eingelassen. Dies hat zur Folge, dass die Kompression nur punktuell ausgeübt wird. Dennoch bleibt ein unzureichender Tragekomfort erhalten.

Alternativ zum komprimierenden Material ist auch eine Art Hebevorrichtung für die weibliche Brust bekannt. Hierbei wird das gesamte Gewebe möglichst weit angehoben, damit die Lymphe selbstständig zurückfließen kann. Durch das Anheben des Gewebes wird die Beweglichkeit des Patienten erheblich eingeschränkt und somit auch der Tragekomfort entscheidend verringert.

Der Erfindung liegt daher die Aufgabe zugrunde, ein Kleidungsstück, insbesondere ein therapeutisches Kleidungsstück, der oben genannten Art anzugeben, welches mittels Drucksträngen die manuelle Lymphdrainage durch tragen des Kleidungsstücks nahezu ersetzt und so den Lymphabfluss entscheidend verbessert. In diesem Fall wird nicht mehr ein kompletter Körperteil von der Kompression in Mittleidenschaft gezogen, sondern nur der Bereich der nach einer Operation diese Unterstützung benötigt.

Bezüglich des Kleidungsstücks wird diese Aufgabe erfindungsgemäß gelöst, indem von der Peripherie aus in Richtung der Lymphknotenregionen verlaufend, eine Anzahl von Drucksträngen eingebracht ist.

Vorteilhafte Ausgestaltungen der Erfindung sind Gegenstand der Unteransprüche.

Die Erfindung geht von der Überlegung aus, dass eine Beeinträchtigung des Tragekomforts sowie die Einschränkung der Bewegungsfähigkeit, durch einen konsequenten Verzicht der flächendeckenden Kompression oder weiträumigen Hub des Gewebes, vermieden werden kann. Da der Rückfluss der Lymphe in das Gewebe aber zumindest verlangsamt werden sollte, muss zur Sicherstellung des Abflusses der Lymphe eine Apparatur in das betreffende Kleidungsstück eingebracht werden. Hierbei ist eine Vorrichtung sinnvoll, die mit geringem Druck und Hub auskommt und so einen entsprechenden Tragekomfort liefert. Dazu werden in das Kleidungsstück Druckstränge eingebracht, die den Abfluss der Lymphe und den Abtransport von Hämatomen aus dem Gewebe entscheidend begünstigen.

Um einen entsprechenden therapeutischen Effekt zu erzielen, werden vorteilhafterweise sechs Druckstränge in jedem zu behandelnden Bereich eingesetzt. Diese sechs Druckstränge werden in den Bereichen der Lymphknoten, in einer Art Abflussstelle, zusammengefasst. Die Druckstränge verlaufen zunächst in der Peripherie nebeneinander und werden schließlich, aus der Peripherie kommend, an einem Punkt im Bereich der Lymphknoten zusammengefasst, beispielsweise im Bereich der Achseln des Patienten.

Für einen besonders flächendeckenden Einsatz sind die Druckstränge idealerweise wellenförmig ausgebildet. Dies trägt beim Tragen des Kleidungsstücks dazu bei, dass die auf der Haut liegenden Druckstränge einen größeren Bereich abdecken und so ein größtmöglicher Teil des Gewebes von den Drucksträngen beansprucht wird. Darüber hinaus initialisiert die wellenförmige Ausgestaltung der Druckstränge ein, durch Bewegung verursachtes, Zusammenziehen und Entspannen der Druckstränge auf der Haut. Dies wirkt sich besonders positiv auf die Entstauung des betreffenden Gewebes aus.

Zur Sicherstellung der Hautverträglichkeit sind die Druckstränge sowie die Abflussstelle vorzugsweise aus Silikon gefertigt. Durch dieses Silikon wird die größtmögliche Hautverträglichkeit erreicht. Das Silikon haftet an der Haut und ermöglicht es so dem Kleidungsstück, die betreffenden Hautpartien bei Bewegung anzuheben. So werden zusätzlich die in dem Bereich liegenden Hautrezeptoren aktiviert.

Die zusammengefassten Druckstränge sind von der Peripherie kommend in einer Abflussstelle zusammengefasst, welche sich unmittelbar über den entsprechenden Lymphknotenregionen befindet. Dabei ist die Abflussstelle vorzugsweise, eine auf der Haut aufliegende Fläche, in der die in diesem Bereich verlaufenden Druckstränge enden. Diese Abflussstelle dient somit als zentraler Abflussort und leitet die Lymphe indirekt zurück in die Lymphknoten.

Speziell nach Operationen, die eine teilweise Amputation beinhalteten, ist eine weitere vorteilhafte Ausführung des therapeutischen Kleidungsstücks als Büstenhalter vorgesehen. Dies hat den Vorteil, eine gegebenenfalls benötigte Tasche für eine Brustprothese oder eine Epithese in den Büstenhalter problemlos einarbeiten zu können.

Eine noch weitergehende Form des therapeutischen Kleidungsstücks stellt die Ausführung als Body dar. Im Gegensatz zum Büstenhalter, ermöglicht der Body vorteilhafterweise die Abführung der Lymphe nicht nur im Bereich des Oberkörpers, sondern schließt auch die Leistenregion mit ein. Hierbei bietet der Body ähnliche Vorteile gegenüber konventionellen Kleidungsstücken wie der bereits erwähnte Büstenhalter. Insgesamt ermöglicht der Body eine großflächigere Rückführung der Lymphe zu den unterschiedlichen Lymphknotenregionen des Oberkörpers, wie zum Beispiel zu den Flanken oder der Bauchregion.

Gemäß dem Fall, dass der Fokus der Rückführung der Lymphe sich nicht auf die Achseln beziehungsweise auf den Hals bezieht, könnte man idealerweise das Kleidungsstück auch als Body ausführen. Diese Maßnahme begünstigt die Einbindung der Lymphknoten in der Bauchregion sowie die in der Leistenregion vorhandenen Lymphknoten. Somit wird der gesamte Oberkörper in den Lymphabtransport mit einbezogen. Dabei kann ein gesteigerter Tragekomfort erreicht werden, da die Halsund Schulterregionen lediglich geringfügige haltende Funktionen übernehmen müssen.

Sofern es vorzuziehen ist, die Schultern zum Rücktransport der Lymphe verstärkt mit einzubeziehen, ist es zweckmäßigerweise auch möglich das Kleidungsstück als eine Art Hemd herzustellen. Dies ermöglicht nicht nur einen verbesserten Halt des Kleidungsstücks am Oberkörper, sondern ermöglicht auch weiterführende Optionen der Kompression des Oberkörpers sowie ein weitläufiger Einsatz der Druckstränge auch in der Bauchregion.

Die mit Erfindung erzielten Vorteile bestehen insbesondere in der Unterstützung des Lymphabtransportes sowie der Verbesserung der Beweglichkeit des Patienten und dessen Wohlbefinden. Bisher wird der Lymphabfluss dadurch unterstützt, dass Kleidungsstücke mittels Kompression des Gewebes, sowie dessen Hub, den Lymphabfluss stimulieren. Die damit einhergehende Beeinträchtigung der Bewegungsfreiheit wird von Patienten als sehr unangenehm empfunden. Dennoch ist während des Heilungsprozesses des Lymphsystems eine Unterstützung des Lymphabflusses unverzichtbar. Dabei wird neben zu tragenden Kleidungsstücken auch durch spezielle Massagetechniken eine Rückführung der Lymphe begünstigt. Um die gewünschten Heilungserfolge zu erzielen, bietet sich hier eine Kombination der manuellen Lymphdrainage und der Kleidungsstücke an. Hierbei wird weniger Druck benötigt, da die vorliegende Erfindung durch Druckstränge zum einen punktuell Druck auf die entsprechenden Stellen ausübt, zum anderen bei jeder Bewegung des Patienten die Haut und das darunter liegende Gewebe in Bewegung versetzt. Dadurch wird der gewünschte Lymphabfluss verstärkt angeregt. Darüber hinaus begünstigt das Kleidungsstück den Abtransport von Blutergüssen beziehungsweise Hämatomen. Diese Eigenschaften sind besonders für die Thorax- und Unfallchirurgie von großer Bedeutung, da durch das Kleidungsstück der Heilungsprozess positiv beeinflusst und das Narbengewebe weicher wird.

Ein Ausführungsbeispiel der Erfindung wird anhand einer Zeichnung näher erläutert. Darin zeigen:
- FIG. 1: Frontansicht eines therapeutischen Kleidungsstücks in Form eines Büstenhalters mit Ableitung in Richtung beider Achseln sowie in Richtung des Schlüsselbeins,
- FIG. 2: Rückansicht mit Ableitung zur rechten Achselhöhle,
- FIG. 3: Rückansicht mit Ableitung zur linken Achselhöhle,
- FIG. 4: Rückansicht mit Ableitung zu beiden Achselhöhlen,
- FIG. 5: Frontansicht mit Ableitung zum Sternum sowie nur einer Ableitung in Richtung der Achselhöhle,
- FIG. 6: Seitenansicht links mit zwei Basen im Bereich der Achselhöhle,
- FIG. 7: Frontansicht mit Seitenschlitz auf der rechten Seite zum Einlegen einer Brustprothese,
- FIG. 8: Frontansicht Body mit Abflussstellen in der Bauch-, Schlüsselbein- und Leistenregion, und
- FIG. 9: beispielhafte Übersicht des weiblichen Lymphsystems.

Gleiche Teile sind in allen Figuren mit denselben Bezugszeichen versehen.

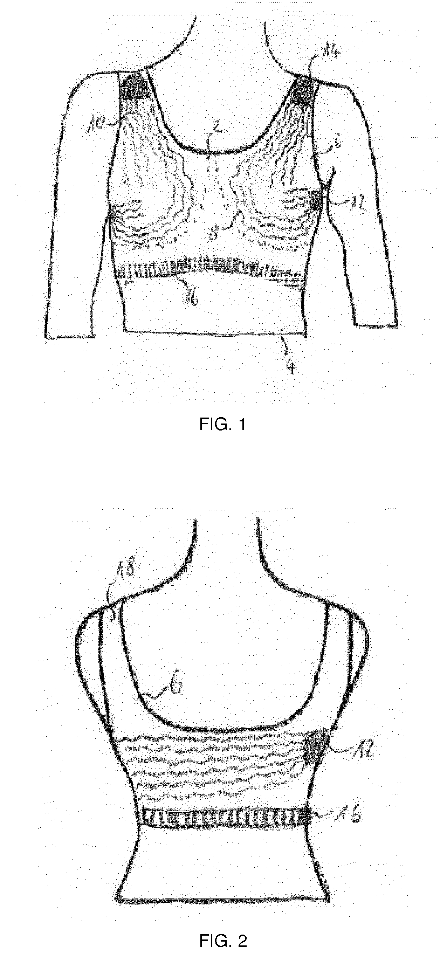

FIG. 1 zeigt eine Frontansicht einer beispielhaften Ausführungsform des angelegten therapeutischen Kleidungsstücks 2 für den Oberkörper 4 in Form eines Büstenhalters 6. Das therapeutische Kleidungsstück 2 umfasst wellenförmige, an der Haut anliegende Druckstränge 8, zwei Abflussstellen im Bereich der Achselhöhle 12, zwei Abflussstellen im Bereich des Schlüsselbeins 14 und den unterstützenden Unterbrustgurt 16. Deutlich zu erkennen ist, wie die wellenförmigen Druckstränge 8 von der Peripherie hin zu den betreffenden Lymphknotenregionen verlaufen. In dieser Ausführungsform sechs wellenförmige Druckstränge 8 gebildet, wobei die wellenförmigen Druckstränge 8 im Bereich der Brustwarze radial um diese herum verlaufen. Darüber hinaus besitzt das therapeutische Kleidungsstück keine störenden Haken oder Ösen und ist im Schulterbereich besonders breit ausgebildet, um einen größtmöglichen Tragekomfort zu gewährleisten, sowie eine sechsfache Ausführung der Druckstränge zu ermöglichen.

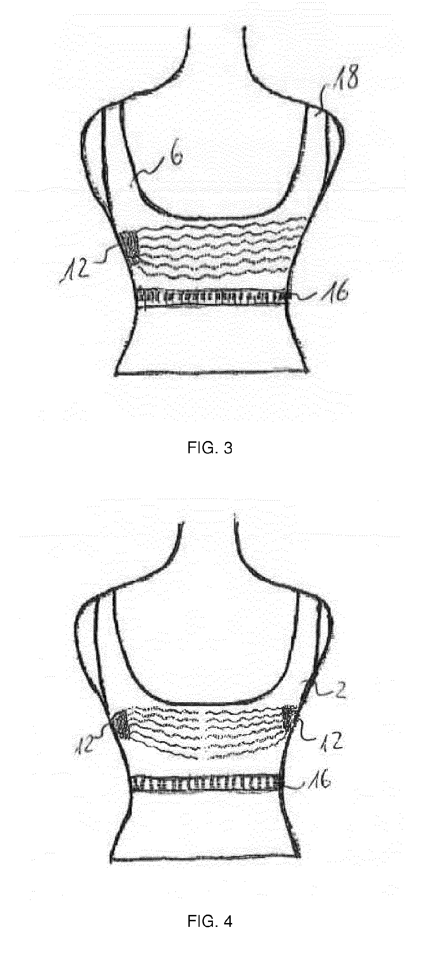

FIG. 2 und FIG. 3 zeigen die Rückansicht eines Büstenhalters 6 mit jeweils versetzten Abflussstellen im Bereich der Achselhöhlen 12. Wobei in FIG. 2 die Ableitung der Lymphe im unteren Brustwirbelbereich über die gesamte Breite des Rückens hin zur rechten Abflussstelle 12 in der Nähe der Achselhöhle angestrebt wird. In FIG. 3 wird die gleiche Apparatur gezeigt, wobei die Ableitung hier zur linken Abflussstelle der Achselhöhle 12 erfolgt. Des Weiteren wird der Büstenhalter 6 durch je ein Haltegurt 18 über die rechte Schulter sowie die linke Schulter und den unterhalb der Brust verlaufenden Unterbrustgurt 16 am Oberkörper fixiert.

FIG. 4 zeigt eine Ausführungsform als Büstenhalter 6 der die gleichmäßige Ableitung in Richtung der rechten und linken Abflussstelle der Achselhöhle 12 in einem therapeutischen Kleidungsstück 2 vereint.

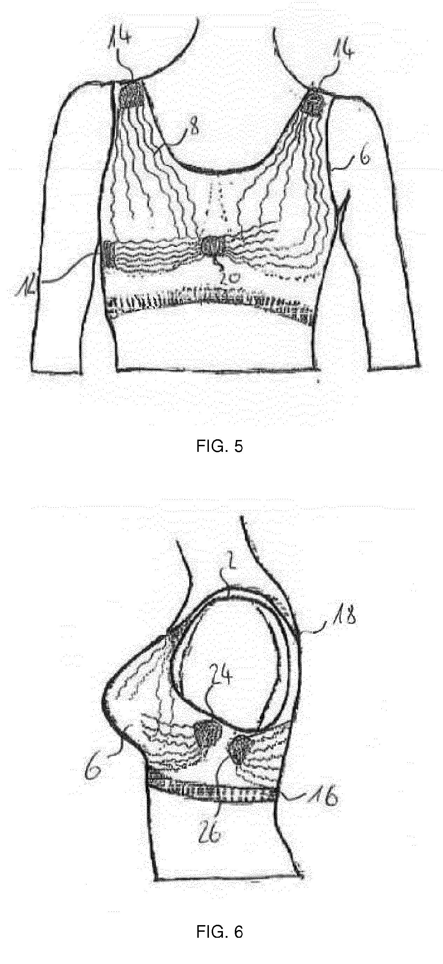

In der in FIG. 5 aufgeführten Variante des Büstenhalters 6 wird die Lymphableitungskonstruktion nun um eine Zwischenbasis 20 im Bereich des Sternums 22 erweitert. Darüber hinaus ist eine Abflussstelle im Bereich der Achselhöhle 12 sichtbar, sowie zwei weitere Abflussstellen im Bereich des Schlüsselbeins 14. Des Weiteren verdeutlich FIG. 5, dass die wellenförmigen Druckstränge 8 nicht zwangsläufig durchgehend sein müssen. Vielmehr ist auch eine Version mit unterbrochenen wellenförmigen Drucksträngen 8 denkbar.

In der in FIG. 6 dargestellten Ausführung des therapeutischen Kleidungsstücks 2 wird eine weitere mögliche Variante eines Büstenhalters 6 dargestellt. In diesem Fall wird eine Ableitung der Lymphe sowohl von vorne aus dem Brustbereich hin zu einer Abflussstelle Brust 24, als auch eine Ableitung des Rücken- und Schulterbereich in Richtung einer Abflussstelle Rücken 26 angestrebt. Der Büstenhalter 6 wird dabei von einem Haltegurt 18 und einem Unterbrustgurt 16 in der gewünschten Position gehalten.

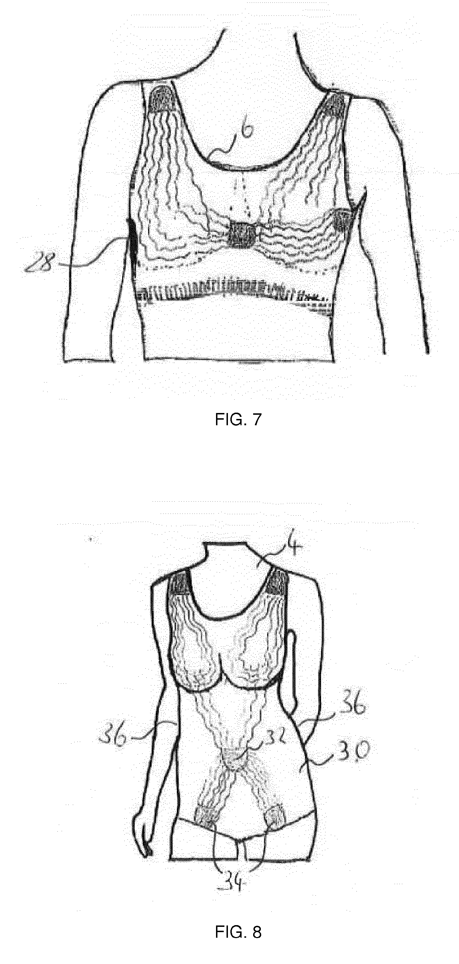

FIG. 7 zeigt eine Ausführungsform des Büstenhalters 6 mit einer seitlichen Öffnung 28 zum Einlegen einer Brustprothese. In diesem Beispiel auf der rechten Seite des Patienten. Alternativ ist auch eine Öffnung linksseitig oder beidseitig möglich.

In FIG. 8 wird eine weitere Ausführungsform des therapeutischen Kleidungsstücks 2 aufgeführt. In diesem Beispiel handelt es sich um eine Art Ganzkörperbody 30. In dieser Form wird eine Abführung der Lymphe im kompletten Oberkörper 4 realisiert. Diese Ausführungsform ermöglicht, neben der Zurückführung der Lymphe im Schulterbereich, auch einen Abfluss der Lymphe in Richtung einer Abflussstelle Bauchregion 32, sowie in die Leistenregionen 34. In dieser Abbildung nicht explizit aufgeführt, jedoch auch möglich, ist die Abführung der Lymphe hin zu der rechten und linken Flanke 36 des Patienten.

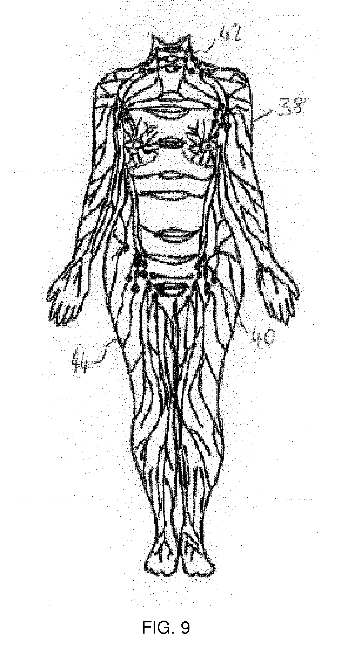

FIG. 9 ist eine beispielhafte Abbildung des weiblichen Lymphsystem. Deutlich zu erkennen sind die Lymphknoten im Bereich der Achseln 38, der Leiste 40 und des Halses 42. Außerdem wird ein exemplarischer Verlauf der Lymphbahnen 44 dargestellt.

### Bezugszeichenliste

- 2: therapeutisches Kleidungsstück
- 4: Oberkörper
- 6: Büstenhalter
- 8: Druckstränge
- 12: Abflussstelle im Bereich der Achselhöhle
- 14: Abflussstelle im Bereich des Schlüsselbeins
- 16: Unterbrustgurt
- 18: Haltegurt
- 20: Zwischenbasis
- 22: Sternum
- 24: Abflussstelle Brust
- 26: Abflussstelle Rücken
- 28: seitliche Öffnung
- 30: Ganzkörperbody
- 32: Abflussstelle Bauchregion
- 34: Abflussstelle Leistenregion
- 36: Flanke
- 38: Lymphknoten Achsel
- 40: Lymphknoten Leiste
- 42: Lymphknoten Hals
- 44: Lymphbahnen

## Patentansprüche

1. Therapeutisches Kleidungsstück (2) zum Tragen auf der Haut, in das auf der der Haut zugewandten Seite, von der Peripherie aus in Richtung der Lymphknotenregionen verlaufend, eine Anzahl von Drucksträngen (8) eingebracht ist.

2. Therapeutisches Kleidungsstück (2) nach Anspruch 1, wobei die Druckstränge (8) wellenförmig ausgebildet sind.

3. Therapeutisches Kleidungsstück (2) nach einem der Ansprüche 1 oder 2, wobei die Druckstränge (8) auf Silikonbasis aufgebaut sind.

4. Therapeutisches Kleidungsstück (2) nach einem der Ansprüche 1 bis 3, wobei die Druckstränge (8) in eine kompakte Abflussstelle (12) zusammengeführt sind.

5. Therapeutisches Kleidungsstück (2) nach einem der Ansprüche 1 bis 4, das in Form eines Büstenhalters (6), eines Ganzkörperbodys (30), eines Bustiers oder eines Hemdes aufgebaut ist.
